Europäisches Patentamt
European Patent Office
Office européen des brevets

(11) Veröffentlichungsnummer: **0 329 613**
**A2**

# (12) EUROPÄISCHE PATENTANMELDUNG

(21) Anmeldenummer: **89810108.4**

(22) Anmeldetag: **08.02.89**

(51) Int. Cl.4: **C 07 D 213/74**
G 02 F 1/35, C 07 C 87/60,
C 07 C 97/00

(30) Priorität: **17.02.88 CH 576/88**

(43) Veröffentlichungstag der Anmeldung:
**23.08.89 Patentblatt 89/34**

(84) Benannte Vertragsstaaten:
**CH DE FR GB IT LI NL**

(71) Anmelder: **CIBA-GEIGY AG**
**Klybeckstrasse 141**
**CH-4002 Basel (CH)**

(72) Erfinder: **Decher, Gero, Dr.**
**Rheinstrasse 9**
**D-6229 Walluf (DE)**

**Tieke, Bernd, Dr.**
**Route Bel-Air 22**
**CH-1723 Marly (CH)**

**Bosshard, Christian**
**Zürcherstrasse 115**
**CH-5400 Baden (CH)**

**Günter, Peter, Prof. Dr.**
**Püntstrasse 17**
**CH-8173 Riedt-Neerach (CH)**

## (54) Organische Materialien mit nichtlinearen optischen Eigenschaften.

(57) Beschrieben wird ein filmförmiges Material enthaltend Verbindungen der Formel I in orientierter, nicht punktsymmetrischer Anordnung

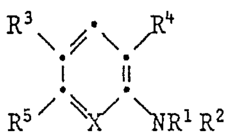

(I),

worin X =CH- oder N- bedeutet,
$R^1$ $C_{12}$-$C_{30}$-Alkyl ist, $R^2$ Wasserstoff oder $C_1$-$C_{30}$-Alkyl ist,
$R^3$ $-NO_2$, -CN, $-CF_3$, $-COCF_3$, $-SO_2CH_3$ oder $-SO_2CF_3$ bedeutet,
$R^4$ Wasserstoff ist oder die gleiche Bedeutung wie $R^3$ besitzt,
$R^5$ Wasserstoff oder $-NR^6R^7$ ist, und $R6^6$und $R^7$ unabhängig voneinander Wasserstoff oder $C_1$-$C_{30}$-Alkyl sind, wobei irgendwelche Alkylreste auch teil- oder perfluoriert sein können.

Die Verbindungen der Formel I lassen sich in Langmuir-Blodgett Schichtsystemen anordnen. Solche Systeme lassen sich beispielsweise zur Herstellung von optoelektronischen Bauelementen einsetzen.

EP 0 329 613 A2

**Beschreibung**

## Organische Materialien mit nichtlinearen optischen Eigenschaften

Die vorliegende Erfindung betrifft organische Materialien in ausgewählter Anordnung, insbesondere in Form von Langmuir-Blodgett Schichtsystemen, ausgewählte Verbindungen, ein Verfahren zur Frequenzverdoppelung sowie die Verwendung dieser Materialien in optoelektronischen Bauteilen.

In nichtlinearen optischen Materialien (NLO-Materialien) werden bekanntermassen elektromagnetische Felder durch Wechselwirkung mit dem Material in neue elektromagnetische Felder mit veränderter Phase, Amplitude, Frequenz und Ausbreitungscharakteristik transformiert.

Solche Materialien haben in den letzten Jahren beträchtliches Interesse als potentielle Komponenten elektrooptischer Bauteile gefunden. In diesen Materialien ist die durch ein elektrisches Feld hervorgerufene Polarisation P eine Funktion der linearen und der nichtlinearen Terme der elektrischen Feldstärke E

$P = \tau^{(1)} \bullet E + \tau^{(2)} \bullet E^2 + \tau^{(3)} \bullet E^3 + .....$ .

Dabei bedeutet $\tau^{(i)}$ (i = 1, 2, 3 ...) die elektrische Suszeptibilität, die wiederum eine Funktion molekularer Parameter und der Anordnung der Moleküle ist. Zur Erzeugung nichtlinearer optischer Effekte zweiter Ordnung muss $\tau^{(2)}$ definitionsgemäss ungleich Null sein. Beispiele für solche Effekte sind die Frequenzverdopplung, die optische Gleichrichtung und der sogenannte Pockels-Effekt.

Es ist bekannt, dass zur Erzeugung von nichtlinearen optischen Effekten zweiter Ordnung geeignete Materialien orientiert sein müssen, aber keine Punktsymmetrie aufweisen dürfen; die einzelnen Moleküle dürfen also keine Punktsymmetrieelemente besitzen und sie dürfen sich auch nicht so anordnen (beispielsweise in einem Kristall), dass dadurch Punktsymmetrie oder Isotropie entsteht.

Eine weitere Bedingung an NLO-Materialien ist die Abwesenheit von starken Absorptionsbanden im Bereich der erzeugenden und der transformierten Strahlung, da durch eine Absorption im Bereich der erzeugenden Strahlung das Material zerstört werden könnte und durch eine Absorption im Bereich der transformierten Strahlung die erzielbaren Effekte verkleinert würden. So besteht insbesondere ein Bedürfnis nach organischen NLO-Materialien mit einer möglichst kurzwelligen Absorption (sogenannte "cut-off Wellenlänge").

Eine Möglichkeit zur gezielten Anordnung von organischen Molekülen ist das Langmuir-Blodgett Verfahren (LB-Verfahren), bei dem monomolekulare Filme aus amphiphilen Verbindungen sukzessive auf feste Substrate übertragen werden.

Weitere Möglichkeiten zur gezielten Anordnung von organischen Molekülen ist die Ausrichtung in Polymerfilmen [vergl. J.D. Williams in "Angewandte Chemie", 96, 637-651 (1984)] oder die Herstellung von Einschlussverbindungen, wie in der JP-A-60-178,427 beschrieben.

Langmuir-Blodgett-Schichtsysteme (LB-Systeme) mit nicht linearen optischen Eigenschaften sind bereits bekannt. Diese Systeme enthalten amphiphile Merocyanine, Hemicyanine oder Azobenzole als aktive Materialien. Beispiele für solche Systeme findet man in J.Opt.Soc.Am., B4(6), 950-4 (1987), Electronics Letters, 22(9), 460-2 (1986), Thin Solid Films, 132, 101-12 (1985), Sov.Techn. Phys.Lett., 11(5), 250 (1985), Opt.Communic., 61(5), 351-6 (1987) und in der EP-A-203,7800.

Es wurden jetzt amphiphile Verbindungen gefunden, die sich in einfacher Weise in Filmen in nicht punktsymmetrischer Weise anordnen lassen, die eine kurze "cut-off" Wellenlänge besitzen und die eine hohe Effektivität bei der Frequenzverdopplung zeigen.

Die vorliegende Erfindung betrifft ein filmförmiges Material enthaltend Verbindungen der Formel I in orientierter, nicht punktsymmetrischer Anordnung

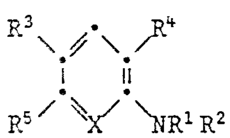

(I),

worin X =CH- oder =N- bedeutet,

$R^1$ $C_{12}$-$C_{30}$-Alkyl ist, $R^2$ Wasserstoff oder $C_1$-$C_{30}$-Alkyl ist,

$R^3$ $-NO_2$, -CN, $-CF_3$, $-COCF_3$, $-SO_2CH_3$ oder $-SO_2CF_3$ bedeutet,

$R^4$ Wasserstoff ist oder die gleiche Bedeutung wie $R^3$ besitzt,

$R^5$ Wasserstoff oder $-NR^6R^7$ ist, und $R^6$ und $R^7$ unabhängig voneinander Wasserstoff oder $C_1$-$C_{30}$-Alkyl sind, wobei irgendwelche Alkylreste auch teil- oder perfluoriert sein können.

Unter dem Begriff "filmförmiges Material enthaltend.....in orientierter, nicht punktsymmetrischer Anordnung" versteht man ganz allgemein jedes filmförmige Material, in dem zumindest ein Teil der Verbindungen der Formel I ausgerichtet ist und in nicht punktsymmetrischer Anordnung fixiert ist. Eine solche Anordnung dieser Verbindungen resultiert in polaren Filmen (makroskopisches Dipolmoment).

Dabei kann es sich um feste oder flüssig-kristalline filmförmige Materialien handeln; ferner kann es sich bei dem Material um eine Mischung enthaltend gelöste oder in Form von Molekülaggregaten vorliegende

Verbindungen der Formel I in einem Trägermaterial handeln oder das Material kann im wesentlichen aus den Verbindungen der Formel I bestehen.

Beispiele für anisotrope Mischungen von Verbindungen der Formel I sind Mischungen in niedermolekularen oder polymeren Materialien, die in der Regel unterhalb ihres Schmelzpunktes bzw. ihrer Glastemperatur vorliegen. Beispiele dafür sind Mischungen ausgerichteter Verbindungen der Formel I in Polymerfilmen (beispielsweise erhältlich durch Orientierung der Verbindungen mittels eines elektrischen Feldes oberhalb der Glastemperatur des Trägerpolymeren und anschliessendes Einfrieren der ausgerichteten Verbindungen durch Abkühlen unter die Glastemperatur; vergl. den oben erwähnten Artikel von D.J. Williams).

Es kann sich aber auch um mikrokristalline Filme aus den Verbindungen der Formel I handeln. Eine NLO-Aktivität wird in diesen Fällen nur dann zu beobachten sein, wenn die Elementarzelle der Mikrokristalle keine Punktsymmetrieelemente aufweist.

Der Zusammenhang zwischen Molekülstruktur und Kristallstruktur ist in der Regel nicht vorhersagbar, so dass es dem Fachmann im Einzelfall überlassen sein muss, die NLO-Eigenschaften eines mikrokristallinen Films aus Verbindungen der Formel I anhand der bekannten NLO-Effekte nachzuprüfen.

Mit Hilfe der LB-Technik lässt sich in der Regel immer eine nicht punktsymmetrische Anordnung der Verbindungen der Formel I erzielen. Die Erfindung betrifft daher vorzugsweise ein Material, worin die Verbindungen der Formel I in Form von Langmuir-Blodgett Schichtsystemen (LB-Systemen) angeordnet sind.

Zur Herstellung dieser LB-Systeme trägt man in an sich bekannter Weise eine geringe Menge einer Lösung einer Verbindung der Formel I in einem niedrigsiedenden Lösungsmittel auf eine freie Wasseroberfläche auf, lässt das Lösungsmittel verdampfen und komprimiert den entstandenen Film, so dass eine stabile, monomolekulare Schicht auf der Wasseroberfläche entsteht.

Die Stabilität der Filme lässt sich bekanntermassen durch Wahl der experimentellen Parameter beeinflussen. So kann man beispielsweise Filme aus relativ kurzkettigen Verbindungen der Formel I durch Kühlen der Subphase stabilisieren oder man verwendet Verbindungen der Formel I in Kombination mit anderen amphiphilen Verbindungen, die zur Ausbildung von stabilen monomolekularen Filmen auf der Wasseroberfläche befähigt sind oder monomolekulare Filme zu stabilisieren vermögen.

Beispiele für solche Verbindungen sind langkettige Carbonsäuren, wie Palmitinsäure, Stearinsäure, Arachinsäure oder Behensäure oder die Ester, insbesondere die Methylester oder die Amide dieser Säuren; langkettige primäre Amine, wie n-Hexadecyl-, n-Octadecyl- oder n-Eicosylamin; langkettige Alkohole, wie n-Hexadecan-, n-Octadecan-oder n-Eicosanol; langkettige Kohlenwasserstoffe, wie Hexadecan, Octadecan oder Eicosan; oder Steroide und Steroidderivate, wie Cholesterol.

Man kann auch amphiphile Verbindungen mit polymerisierbaren Gruppen verwenden; Beispiele dafür sind ω-Tricosensäure, Diacetylencarbonsäuren oder langkettige Sorbinsäureester. Ferner können auch filmbildende Polymere verwendet werden, die auf der Wasseroberfläche gespreitet werden und anschliessend übertragen werden; ein Beispiel dafür ist Poly-Stearoylhydroxyethylmethacrylat.

Die Verbindungen der Formel I sind in der Regel zu wenigstens 1 Gew.%, bezogen auf die Gesamtmischung, in solchen Mischungen enthalten.

Monomolekulare Schichten auf der Wasseroberfläche lassen sich nach dem Langmuir-Blodgett Verfahren (LB-Verfahren) auf feste Träger übertragen. Dazu taucht man in an sich bekannter Weise einen festen Träger mit im wesentlichen glatter Oberfläche durch einen komprimierten, monomolekularen Film auf einer Wasseroberfläche und überträgt dadurch besagten Film auf den Träger.

Durch mehrmaliges Ein- und Austauchen lassen sich auf diese Weise Mehrschichtsysteme herstellen.

Die Schicht auf der Wasseroberfläche kann nach jedem Tauchvorgang ausgetauscht werden, so dass unterschiedliche Folgen von Schichten auf dem Träger herstellbar sind.

Je nach Hydrophilie oder Hydrophobie der Trägeroberfläche wird der Träger beim ersten Eintauchvorgang (hydrophober Träger) oder Austauchvorgang (hydrophiler Träger) beschichtet. Bei hydrophilen Trägern kommen die polaren Gruppen mit der Trägeroberfläche in Kontakt, während bei hydrophoben Trägern die hydrophoben Reste $R^1$ und $R^2$ mit der Trägeroberfläche in Kontakt kommen. Diese Verfahrensweisen sind dem Fachmann auf dem Gebiet der LB-Systeme an sich bekannt.

Als feste Träger für das LB-Verfahren kommen unterschiedlichste Substrate mit mikroskopisch planarer Oberfläche in Frage. Beispiele dafür sind Metalle, wie Aluminium, Kupfer, Silber oder Gold, Halbleiter, wie Germanium, Silizium oder GaAs, anorganische Materialien, wie Glas, Quarz, $KNbO_3$, $LiNbO_3$, ZnSe oder $Si_2N_3$ oder Kunststoffe, wie Teflon®, Polyethylen, Polypropylen, Polymethylmethacrylat oder Polyester. Man kann auch hydrophobisierte Träger benutzen, beispielsweise Glas oder Quarz, das mit Trichlormethylsilan, Dichlordimethylsilan oder Trichloroctadecylsilan vorbehandelt wurde.

Die Subphase, auf der die monomolekulare Schicht ausgebildet wird, besteht in der Regel aus mehrfach destilliertem Wasser, dem gegebenenfalls zur Stabilisierung der Filme geringe Mengen von Salzen, beispielsweise von $CdCl_2$ oder $BaCl_2$, zugesetzt sind. Ferner kann die Subphase Puffersubstanzen, beispielsweise $NaHCO_3$, enthalten. Diese Modifikationen sind dem Fachmann bekannt und werden je nach der Art der filmbildenden Substanzen ausgewählt.

Aus den reinen Farbstoffmonoschichten oder aus Mischfilmen enthaltend Verbindungen der Formel I und weitere filmbildende Materialien lassen sich Multischichten nach dem LB-Verfahren herstellen. Diese Schichten können unmittelbar aufeinander folgen oder es handelt sich um alternierende Schichten aus Farbstoffmonoschichten und anderen schichtbildenden, nicht unbedingt NLO-aktiven Materialien.

Durch Uebertragung der Monoschichten können Multischichtsysteme des X-, Y- oder Z-Typs aufgebaut

werden. Diese Multischichtsysteme sind dem Fachmann an sich bekannt und beispielsweise in "Techniques of Chemistry, Physical Methods of Chemistry, Vol. I, Part IIIB, S. 666-669", beschrieben.

Dabei können jeweils Filme aus ein und demselben NLO-aktiven Material (Filme enthaltend Verbindungen der Formel I) übertragen werden oder man überträgt unterschiedliche Filme und stellt auf diese Weise alternierende Schichtfolgen her.

Bei den alternierenden Schichtfolgen bevorzugt man folgende drei Varianten:

a) Beim Eintauchen wird ein NLO-aktiver Film bzw. ein NLO-aktiver Mischfilm übertragen und beim Austauchen wird ein NLO-inaktiver Film bzw. ein NLO-inaktiver Mischfilm übertragen.

b) Beim Eintauchen wird ein NLO-inaktiver Film bzw. Mischfilm übertragen und beim Austauchen wird ein NLO-aktiver Film oder Mischfilm übertragen.

c) Beim Ein- und Austauchen werden jeweils NLO-aktive Filme oder Mischfilme übertragen, wobei diese Filme abwechselnd Verbindungen der Formel I mit unterschiedlich gerichteten Dipolmomenten enthalten.

Die Dicke der erfindungsgemässen LB-Schichtsysteme bewegt sich in der Regel im Bereich von 3 nm bis 5 µm.

Bevorzugt werden LB-Systeme aus alternierenden oder nichtalternierenden Schichten enthaltend als NLO-aktives Material eine Verbindung der Formel I, worin X =CH- ist, $R^1$ $C_{18}$-$C_{22}$-Alkyl ist, $R^2$ Wasserstoff oder $C_{18}$-$C_{22}$-Alkyl bedeutet, $R^3$ -$NO_2$ ist, $R^4$ Wasserstoff oder -$NO_2$ bedeutet und $R^5$ Wasserstoff oder -$NH_2$ ist.

Bevorzugt werden LB-Systeme enthaltend als NLO-aktives Material eine Verbindung der Formel I, worin X =N- ist.

Systeme aus diesen Verbindungen zeichnen sich durch eine besonders hohe NLO-Aktivität aus.

Ganz besonders bevorzugt werden LB-Systeme enthaltend alternierende oder bevorzugt nichtalternierende Schichten aus Verbindungen der Formel I, worin X =N- ist, $R^1$ $C_{16}$-$C_{26}$-Alkyl ist, $R^2$ Wasserstoff bedeutet, $R^3$ -$NO_2$ ist, $R^4$ Wasserstoff oder -$NO_2$ bedeutet und $R^5$ Wasserstoff ist; ganz besonders bevorzugt in dieser Ausführungsform wird das Derivat der Formel I, worin $R^1$ $C_{22}$-Alkyl ist.

Die Verbindungen der Formel I, worin X =N- ist, sind neu und stellen ebenfalls einen Gegenstand der vorliegenden Erfindung dar. Bedeuten irgendwelche Reste Alkyl, so kann es sich dabei um geradkettige oder verzweigte Reste handeln.

Bevorzugt werden geradkettige Reste.

Ferner können die Alkylreste teil- oder perfluoriert sein oder sie können eine oder mehrere trans-Doppelbindungen oder Dreifachbindungen aufweisen, die nicht in 1-Position stehen. Mehrere trans-Doppelbindungen oder Dreifachbindungen können isoliert oder miteinander konjugiert sein.

Spezifische Beispiele für Alkylreste sind Methyl, Ethyl, n-Propyl, Isopropyl, n-Butyl, Isobutyl, n-Pentyl, n-Hexyl, n-Heptyl, n-Octyl, 2-Ethylhexyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Heneicosyl, n-Docosyl, n-Tetracosyl, n-Hexacosyl, n-Octacosyl und n-Triacontyl, sowie die entsprechenden perfluorierten Derivate oder Allyl, Propargyl, Octadec-9-trans-enyl, ω-Tricosenyl oder Pentacosa-10,12-diinyl.

Bei $R^1$ und $R^2$ handelt es sich bevorzugt um Reste der Formel -$C_pH_{2p+1}$, worin p 12-30, bevorzugt 16-26, ganz besonders bevorzugt 20-24 ist. Solche Reste sind bevorzugt geradkettig.

$R^1$ und $R^2$ müssen eine gewisse Mindestlänge aufweisen, damit sich aus Verbindungen der Formel I auf der Wasseroberfläche stabile monomolekulare Filme ausbilden lassen. Handelt es sich bei $R^2$ um Wasserstoff oder um einen kurzkettigen Alkylrest mit weniger als 12 C-Atomen, so sollte $R^1$ wenigstens 18 C-Atome aufweisen.

$R^3$ ist vorzugsweise -$NO_2$ oder -$COCF_3$; besonders bevorzugt -$NO_2$.

$R^4$ ist vorzugsweise Wasserstoff oder -$NO_2$.

Die Verbindungen der Formel I lassen sich durch nukleophilen Austausch von Halogenatomen gegen Aminogruppen aus den entsprechenden Fluor- oder Chlorbenzolderivaten bzw. den entsprechenden Fluor-oder Chlorpyridinderivaten durch Umsetzung mit den entsprechenden Aminen herstellen.

Beispiele für solche Reaktionen an Fluorbenzolderivaten mit elektronenziehenden Substituenten findet man in "Chromatographia, 14(5), 289-95 (1981)".

Austauschreaktionen an 2-Halogendinitropyridinen sind in "Bull.Acad. Polon.Ser.Sci.Chim., XVI(1), 7-12 (1968)" beschrieben.

Die Wahl der Reaktionsbedingungen bei den nukleophilen Austauschreaktionen ist an sich bekannt. In der Regel legt man dabei die aromatische Halogenverbindung in einem inerten organischen Lösungsmittel gelöst vor und tropft das Amin zu oder man arbeitet unter Druck, beispielsweise bei der Umsetzung der aromatischen Halogenverbindung mit Ammoniak.

Eine Auswahl möglicher Reaktionsparameter findet man in "Methoden der organischen Chemie (Houben-Weyl), Bd. XI/1, S. 24-36, G. Thieme Verlag, Stuttgart (1957)".

Eine Uebersicht über die Wahl der Reaktionsbedingungen im Falle der Pyridinderivate findet man in "The Chemistry of Heterocyclic Compounds: Pyridine and its Derivatives, Part III, Vol. IX, p. 5-7".

Die Amine, die bei den nukleophilen Austauschreaktionen eingesetzt werden, sind an sich bekannt oder lassen sich anhand bekannter Verfahren herstellen.

Primäre Amine erhält man beispielsweise durch Alkylierung von Phthalsäureimiden gefolgt von der Hydrolyse zu den Aminen. Sekundäre Amine erhält man beispielsweise durch Reduktion von einfach

N-alkylierten Amiden mit $LiAlH_4$.

Die Halogennitrobenzol- bzw. Halogennitropyridinausgangsprodukte für die nukleophilen Austauschreaktionen sind teilweise bekannt und im Handel erhältlich. Solche Verbindungen lassen sich durch Einführung von ein oder zwei Nitrogruppen in die entsprechenden Halogenbenzole bzw. Halogenpyridine in an sich bekannter Weise herstellen.

Die Halogen-trifluormethylbenzol-, Halogen-trifluormethylpyridin-, Halogencyanobenzol- und Halogencyanopyridinausgangsprodukte lassen sich aus den entsprechenden Halogencarbonsäurederivaten durch Transformation der -COOH Gruppe(n) in $-CF_3$ oder in -CN Reste herstellen. Solche Umwandlungen sind an sich bekannt.

Die Umwandlung der -COOH Gruppe(n) in $-CF_3$Reste lässt sich beispielsweise durch Umsetzung mit $SF_4$ durchführen. Solche Reaktionen findet man beispielsweise in "Methoden der organischen Chemie (Houben-Weyl), Bd. V/3, S. 88-93, G. Thieme Verlag, Stuttgart (1962)".

Die Umwandlung einer -COOH Gruppe in einen -CN Rest lässt sich in an sich bekannter Weise durch Ueberführung der Carboxylgruppe in das Carbonsäureamid und anschliessende Reduktion zum Nitril bewerkstelligen. Die Halogen-trifluormethylcarbonylbenzol- oder Halogen-trifluormethylcarbonylpyridinausgangsprodukte sind teilweise bekannt oder können nach an sich bekannten Verfahren erhalten werden.

So lässt sich die $CF_3$-CO-Gruppe beispielsweise durch Friedel-Crafts Acylierung der zugrundeliegenden Halogenbenzol- oder Halogenpyridinderivate mittels $(CF_3\text{-}CO)_2O$ einführen.

Ebenso lassen sich die Halogen-trifluormethylsulfonylbenzol- oder Halogen-trifluormethylsulfonylpyridinausgangsprodukte erhalten. Dazu setzt man beispielsweise die zugrundeliegenden Halogenbenzol- oder Halogenpyridinderivate mit $(CF_3\text{-}SO_2)_2O$ um. Solche Reaktionen werden in J.Org.Chem., 42(24), 3875-7 (1977) beschrieben.

Die Halogen-methylsulfonylbenzol- oder Halogen-methylsulfonylpyridinausgangsprodukte lassen sich aus den zugrundeliegenden Halogenbenzolen oder Halogenpyridinen durch Friedel-Crafts Acylierung mit $SO_2$, Aussalzen des gebildeten Sulfinsäurederivates mit NaCl und anschliessende Methylierung des Sulfinsäurenatriumsalzes mit Methylhalogeniden oder mit Dimethylsulfat erhalten. Solche Umsetzungen sind beispielsweise in "Methoden der organischen Chemie (Houben-Weyl), Bd. IX, S. 231-3 und 324-5, G. Thieme Verlag, Stuttgart (1955)" beschrieben.

Neben den nukleophilen Austauschreaktionen lassen sich die Verbindungen der Formel I auch durch Einführung der Reste $R^3$ und gegebenenfalls $R^4$ in die zugrundeliegenden Aminoderivate herstellen. Auf diese Weise lassen sich beispielsweise 3,5-Dinitro-2,6-diaminopyridine erhalten. Solche Reaktionen sind beispielsweise aus der US-A-3,943,125 bekannt.

Auch die weiter oben beschriebenen Umformungen von -COOH in $-CF_3$ oder -CN bzw. die Einführungen von $-CO\text{-}CF_3$, $-SO_2\text{-}CF_3$ oder $-SO_2\text{-}CH_3$ in den zugrundeliegenden Aminocarbonsäurebenzol- oder -pyridinderivaten lassen sich zur Herstellung der Verbindungen der Formel I verwenden.

Die Verbindungen der Formel I lassen sich zur Herstellung optoelektronischer Bauelemente einsetzen. Bevorzugte Einsatzgebiete sind die Verwendung als Frequenzverdoppler oder als Wellenleiter.

Die Erfindung betrifft auch die Verwendung der Verbindungen der Formel I für diese Zwecke.

Die folgenden Beispiele erläutern die Erfindung.

A) Herstellungsbeispiele

Beispiel 1: Synthese von 2-Hexadecylamino-5-nitropyridin 1

0,5 g (3,2 mmol) 2-Chlor-5-nitropyridin (Fluka; purum), 0,76 g (3,2 mmol) Hexadecylamin (Merck-Schuchard; zur Synthese) und 1 g $NaHCO_3$ werden eingewogen und in 10 ml $CHCl_3$/DMF 1:1 (puriss.,p.a.) aufgeschlämmt. Es wird auf 100°C (Badtemperatur) erwärmt und 4 Stunden bei dieser Temperatur gerührt. Nach Abkühlung des Reaktionsgemisches wird mit Wasser versetzt und mit Diethylether extrahiert. Die organische Phase wird zweimal mit Wasser extrahiert und mit $Na_2SO_4$ getrocknet. Nach Verdampfen des Lösungsmittels wird über die 50-fache Menge Kieselgel chromatographiert (Laufmittel Hexan/Ether 95:5). Die leicht gelbliche Produktfraktion wird eingeengt und 1 nochmals aus Hexan/Ether umkristallisiert.

Man erhält 0,94 g gelblicher Kristalle (81 %) vom Schmelzpunkt 76 bis 78°C.

Die elementaranalytischen Daten von 1 sind in Tabelle 1 aufgelistet.

Beispiele 2-6: Synthese von 2-Octadecylamino-5-nitropyridin 2, 2-Eicosylamino-5-nitropyridin 3, 2-Docosylamino-5-nitropyridin 4, 2-Tetracosylamino-5-nitropyridin 5 und 2-Hexacosylamino-5-nitropyridin 6

Bei der Synthese wird im wesentlichen wie unter Beispiel 1 beschrieben verfahren.

Octadecylamin (Fluka) wird vor der Synthese von 2 mehrfach aus Ethanol umkristallisiert.

Eicosylamin, Docosylamin, Tetracosylamin und Hexacosylamin sind literaturbekannt, aber nicht käuflich. Sie werden nach Standardmethoden aus den entsprechenden Carbonsäuren hergestellt (Ueberführung der Carbonsäure in das Säurechlorid mit $SOCl_2$, Umsetzung mit $NH_3$ in Tetra hydrofuran zum Säureamid, Reduktion mit $LiAlH_4$ in Tetrahydrofuran zum Amin). Zur Reinigung der rohen Amine wird mehrfach aus Ethanol umkristallisiert.

Da die Löslichkeit der 2-Alkylamino-5-nitropyridine in Hexan/Ether mit zunehmender Länge der Alkylkette stark abnimmt, werden die Verbindungen 3 bis 6 nicht mehr chromatographisch gereinigt. In diesen Fällen werden die Rohprodukte durch mehrfaches Umkristallisieren aus Ethanol gereinigt. Die Ausbeuten an

Endprodukt liegen in allen Fällen zwischen 60 und 80 %.
Die elementaranalytischen Daten und die Schmelzpunkte von 2-6 sind in Tabelle 1 aufgelistet.

Beispiel 7: Synthese von 2-Octadecylamino-3,5-dinitropyridin 7

Die Synthese erfolgt analog zu Beispiel 1, wobei von 2-Chlor-3,5-dinitropyridin (Fluka) und Octadecylamin ausgegangen wird. Octadecylamin wird vor der Synthese mehrfach aus Ethanol umkristallisiert.
Man erhält nach Chromatographie 0,64 g gelbliche Kristalle (46 % d.Th.) vom Schmelzpunkt 74-77°C. Die elementaranalytischen Daten von 7 sind in Tabelle 2 aufgelistet.

Beispiel 8: Synthese von 2-N,N-(Bis)octadecylamino-5-nitropyridin 8

Die Synthese erfolgt analog zu Beispiel 1, wobei von 2-Chlor-5-nitropyridin (Fluka) und N,N-(Bis)octadecylamin (Fluka 97 %) ausgegangen wird.
Man erhält nach Chromatographie 1,70 g gelbe Nristalle (88 % d.Th.) vom Schmelzpunkt 65-68°C. Die elementaranalytischen Daten von 8 sind in Tabelle 2 aufgelistet.

Beispiel 9: Synthese von 2-N,N-(Bis)octadecylamino-3,5-dinitropyridin 9

Die Synthese erfolgt analog zu Beispiel 1, wobei von 2-Chlor-3,5-dinitropyridin (Fluka) und N,N-(Bis)octadecylamin (Fluka, 97 %) ausgegangen wird.
Man erhält nach Chromatographie 0,96 g gelbe Kristalle (43 % d.Th.) vom Schmelzpunkt 36-41°C. Die elementaranalytischen Daten von 9 sind in Tabelle 2 aufgelistet.

Beispiel 10: 2-N-Methyl-docosylamino-5-nitropyridin 10

Man arbeitet wie unter den Beispielen 2-6 beschrieben und setzt 2-Chlor-5-nitropyridin mit N-Methyl-docosylamin um. Die Ausbeute beträgt 69 % d.Th. Das Produkt besitzt einen Schmelzpunkt von 88-90°C. Die elementaranalytischen Daten von 10 sind in Tabelle 2 aufgelistet.

Beispiel 11: Synthese von 4-Nitro-N-octadecylanilin 11

Die Synthese erfolgt analog zu Beispiel 1, wobei von 1-Fluor-4-nitrobenzol (Fluka purum) und Octadecylamin (Fluka) ausgegangen wird. Octadecylamin wird vor der Synthese mehrfach aus Ethanol umkristallisiert.
Man erhält 0,78 g (62 % d.Th.) gelbe Kristalle vom Schmelzpunkt 78-80°C. Die elementaranalytischen Daten von 11 sind in Tabelle 3 aufgelistet.

Beispiel 12: Synthese von 2,4-Dinitro-N-octadecylanilin 12

Die Synthese erfolgt analog zu Beispiel 1, wobei von 2,4-Dinitrofluorbenzol (Fluka puriss.p.a.) und Octadecylamin (Fluka) ausgegangen wird.
Man erhält nach Chromatographie 0,93 g hellgelbe Kristalle (66 % d.Th.) vom Schmelzpunkt 58-59°C. Die elementaranalytischen Daten von 12 sind in Tabelle 3 aufgelistet.

Beispiel 13: Synthese von 2,4-Dinitro-N,N-(bis)octadecylanilin 13

Die Synthese erfolgt analog zu Beispiel 1, wobei von 2,4-Dinitrofluorbenzol (Fluka puriss.p.a.) und N,N-(Bis)octadecylamin (Fluka 97 %) ausgegangen wird.
Man erhält nach Chromatographie 1,12 g gelbe Kristalle (50 % d.Th.) vom Schmelzpunkt 40-42°C. Die elementaranalytischen Daten von 13 sind in Tabelle 3 aufgelistet.

Beispiel 14-15: Synthese von 2,4-Dinitro-5-amino-N-octadecylanilin 14 und 2,4-Dinitro-5-amino-N-docosylanilin 15

Die Synthese erfolgt analog zu Beispiel 1, wobei von 2,4-Dinitro-5-fluor-anilin (Fluka puriss.p.a.) und Octadecylamin (Fluka) bzw. Docosylamin (s. Beispiel 2-6) ausgegangen wird. Die Extraktion der Rohprodukte erfolgt mit Chloroform anstelle von Ether.
Man erhält nach Umkristallisation aus Ethanol 1,18 g 14 (82 % d.Th.) bzw. 1,37 g 15 (85 % d.Th.) in Form gelblicher Kristalle. Die charakteristischen Daten sind in Tabelle 3 aufgelistet.

Beispiel 16: Synthese von 2,4-Dinitro-5-amino-N,N-(bis)octadecyl anilin 16

Die Synthese erfolgt analog zu Beispiel 1, wobei von 2,4-Dinitro-5-fluoranilin (Fluka puriss.p.a.) und N,N-(Bis)octadecylamin (Fluka 97 %) ausgegangen wird.
Man erhält 1,65 g (71 % d.Th.) einer gelborangen wachsartigen Substanz vom Schmelzpunkt 53-56°C. Die elementaranalytischen Daten von 16 sind in Tabelle 3 aufgelistet.

Beispiel 17: 2,4-Dinitro-5-amino-N-methyl-N-docosyl-anilin 17

Man arbeitet wie unter den Beispielen 14-15 beschrieben und setzt 2,4-Dinitro-5-fluor-anilin mit N-Methyl-docosylamin um. Die Ausbeute beträgt 48 % d.Th. Das Produkt besitzt einen Schmelzpunkt von 87-89°C. Die elementaranalytischen Daten von 17 sind in Tabelle 3 aufgelistet.

Beispiel 18: 4-Trifluoracetyl-N-octadecylanilin 18

Die Synthese erfolgt analog zu Beispiel 1, wobei von 4-Fluortrifluoracetophenon (hergestellt nach J.Org.Chem. 32 (1967) 1311) und Octadecylamin (Fluka) ausgegangen wird. Octadecylamin wird vor der Synthese mehrfach aus Ethanol umkristallisiert. Anstelle des Lösungsmittelgemisches $CHCl_3$/DMF (1:1) wird bei der Synthese reines DMF verwendet.

Das Rohprodukt (1,05 g) wird zweimal aus Ethanol umkristallisiert und über Kieselgel filtriert (Ether/Hexan). Man erhält 0,47 g farblose bis leicht gelbliche Kristalle vom Schmelzpunkt 73-75° C. Analyse:

Analyse:

| | | | | |
|---|---|---|---|---|
| Berechnet für $C_{26}H_{42}NOF_3$ (441.63) | : | C 70,71 %, | H 9,59 %, | N 3,17 %, |
| Gefunden | : | C 70,93 %, | H 9,77 %, | N 3,14 %. |

Beispiel 19: 4-Trifluoracetyl-H-docosylanilin 19

Die Synthese erfolgt analog zu Beispiel 18, wobei als Amin Docosylamin eingesetzt wird. Man erhält 0,57 g farblose bis leicht gelbliche Kristalle vom Schmelzpunkt 75-77° C.

Analyse:

| | | | | |
|---|---|---|---|---|
| Berechnet für $C_{30}H_{50}NOF_3$ (497,73) | : | C 72,39 %, | H 10,13 %, | N 2,81 %, |
| Gefunden | : | C 72,34 %, | H 10,05 %, | N 2,78 %. |

Beispiel 20: 4-Trifluoracetyl-N-tetracosylanilin 20

Die Synthese erfolgt analog zu Beispiel 18, wobei als Amin Tetracosylamin eingesetzt wird. Man erhält 0,32 g farblose bis leicht gelbliche Kristalle vom Schmelzpunkt 89-90° C.

| | | | | |
|---|---|---|---|---|
| Berechnet für $C_{32}H_{54}NOF_3$ (525.78) | : | 73,10 % C, | 10,35 % H, | 2,66 % N |
| Gefunden | : | 72,97 % C, | 10,30 % H, | 2,61 % N |

B) Herstellung von Mono- und Multischichten nach der LB-Technik

Beispiel 21: Herstellung von Monoschichten an der Gas/Wasser-Grenzfläche

Zur Herstellung von Monoschichten auf der Gas/Wasser-Grenzfläche wird eine präparative Lauda-Filmwaage benutzt. Das benötigte hochreine Wasser wird frisch mit einer Milli-Q-Anlage der Firma Millipore hergestellt. Der monomolekulare Film wird durch Auftropfen einer frischen Spreitlösung (Konzentration des Amphiphils in $CHCl_3$ (Merck-Uvasol) 1 ± 0,5 mg/ml) auf die Wasseroberfläche nach Standardtechniken hergestellt. Jeweils nach Verdampfen des Lösungsmittels werden Schub/Flächendiagramme bei verschiedenen Temperaturen registriert (Kompressionsgeschwindigkeit 12,3 $cm^2$/Min.). Als charakteristische Daten werden Kollapsdruck $F_c$ und Kollapsfläche $A_c$ der Monoschicht bei der jeweiligen Temperatur ermittelt (s.Tab. 4).

Beispiel 22: Herstellung von Monoschichten aus Amphiphil-Gemischen an der Gas-Wasser-Grenzfläche

Zur Herstellung der Monoschichten aus Amphiphil-Gemischen wird wie unter Beispiel 21 verfahren. Als Spreitlösung wird eine gemeinsame Lösung von zwei oder mehr Amphiphilen in definierter Zusammensetzung verwendet (Gesamtkonzentration der Amphiphilen in $CHCl_3$ (Merck-Uvasol) 1 ± 0,5 mg/ml). Zusammensetzung und charakteristische Daten dieser Monoschichten sind in Tabelle 5 aufgelistet.

Beispiel 23: Herstellung von LB-Multischichten aus reinen Amphiphilen

Auf einer Subphase aus reinem Wasser der Temperatur T wird eine monomolekulare Schicht analog Beispiel 21 hergestellt und bis zum Filmdruck $F_t$ komprimiert. T und $F_t$ werden für jede Substanz so optimiert, dass die Monoschicht in einer stabilen, kondensierten Phase vorliegt. Anschliessend wird ein fester Träger senkrecht durch die Monoschicht in die Subphase ein- und wieder ausgetaucht mit der Tauchgeschwindigkeit $s_1$ Danach wird der Träger noch mehrfach mit der Tauchgeschwindigkeit $s_2$ ein-und wieder ausgetaucht, wobei $s_2 = 2s_1$ bis $3s_1$ beträgt. Bei jedem Tauchvorgang wird eine Monoschicht übertragen und so bei mehrfachem Tauchen die Multischicht aufgebaut. Die verwendeten Substrate sowie alle charakteristischen Daten sind in Tabelle 6

aufgelistet. Aus den $d_{001}$-Werten folgt, dass die Multischichten in allen Beispielen Y-Struktur besitzen.

Beispiel 24: Herstellung von LB-Multischichten vom X-Typ

Auf einer Subphase aus reinem Wasser der Temperatur 20°C wird eine monomolekulare Schicht der Verbindung 14 analog Beispiel 21 auf einer NIMA-Filmwaage für alternierende Schichten hergestellt und bis zum Filmdruck von 30 mN/m komprimiert. Anschliessend wird ein mit Octadecyltrichlorsilan hydrophobisierter Glasträger mit einer Geschwindigkeit von 10 mm/Min. senkrecht durch die Monoschicht in die Subphase getaucht.

Der Träger wird durch eine reine Wasseroberfläche ausgetaucht, sodass nur beim Eintauchen eine Monoschicht übertragen werden kann. Der Prozess wird 20mal durchgeführt. Es entsteht eine Multischicht vom X-Typ aus 20 Monoschichten Dicke. Die Multischicht ist transparent und besitzt eine optische Dichte von 0,0058 pro Monoschicht (bei $\lambda_{max}$ = 422 nm).

Beispiel 25: Herstellung von LB-Multischichten aus Amphiphil-Gemischen

Auf einer Subphase aus reinem Wasser der Temperatur T wird eine monomolekulare Schicht analog Beispiel 22 gespreitet und bis zum Filmdruck $F_t$ komprimiert. T und $F_t$ werden für jede Substanz so optimiert, dass die Monoschicht in einer stabilen, kondensierten Phase vorliegt. Zur Filmübertragung wird wie bei Beispiel 23 verfahren. Die verwendeten Substrate sowie alle charakteristischen Daten sind in Tabelle 7 aufgelistet.

Beispiel 26: Herstellung von alternierenden LB-Multischichten

Auf einer Subphase aus reinem Wasser der Temperatur T wird eine monomolekulare Schicht der Verbindung 14 analog Beispiel 21 hergestellt und bis zum Filmdruck 35 mN/m komprimiert. Anschliessend wird ein mit Octadecyltrichlorsilan hydrophobisierter Glasträger senkrecht durch die Monoschicht in die Subphase eingetaucht mit der Tauchgeschwindigkeit 10 mm/Min. Nun wird die monomolekulare Schicht von der Oberfläche abgesaugt und eine neue Monoschicht aus Arachinsäure gespreitet und bis zum Filmdruck 35 mN/m komprimiert. Der Glasträger wird jetzt wieder ausgetaucht mit einer Geschwindigkeit von 2,5 mm/Min. Nach Entfernen der Arachinsäuremonoschicht und vollständigem Trocknen des unteren Trägerrandes ($\leqq$ 60 Min. Trockenzeit) wird der beschriebene Vorgang noch fünfmal wiederholt. Es entsteht eine alternierende Multischicht einer Gesamtzahl von 12 Monoschichten Dicke. Die Multischicht ist transparent und besitzt eine optische Dichte von 0,0078 pro alternierender Doppelschicht (bei $\lambda_{max}$ = 417nm).

Beispiel 27: Messung der Frequenzverdopplung in LB-Multischichten

Silanisierte Glasträger werden analog Beispiel 23 mit den Substanzen 2-6 in verschiedener Schichtzahl (vergl. Tab. 8 und 9) beschichtet. Die LB-Filme werden anschliessend mit einem Nd-YAG-Laser (Quatronix 416, 2,3 kW bei 1064 nm, Pulsrate 500 Hz, Pulsdauer 350 ns, Q-switched) in Richtung der Schichtnormalen bestrahlt. Der Laser wird auf den LB-Film mit einer Kollimationslinse (Fokuslänge 80 mm) fokussiert. Der Strahl durchmesser beträgt ungefähr 21 µm (abgeschätzte Intensität: 220 µW/cm$^2$). Das Signal der zweiten harmonischen Oberwelle ($\lambda$ = 532 nm) wird nach der von J.C. Baumert, J. Hoffnagle, P. Günter; Europ. Conf. on Opt. Syst. and Appl.; Amsterdam 1984, SPIE Vol. 492, S. 374 ff. beschriebenen Methode detektiert. Die Ergebnisse findet man in den Tabellen 8 und 9.

Bei allen LB-Filmen wird ein Signal gemessen. $I^{2\omega}$ nimmt quadratisch mit der Schichtzahl zu (s. Tab. 9). Bei der Probe mit 270 Doppelschichten aus 4 liegt die Konversionsrate $I^{2\omega}/I^{\omega}$ bei $2 \cdot 10^{-5}$ % für den Lichtvektor parallel zur Tauchrichtung und $I^{2\omega}$=220 µW/cm$^2$

Beispiel 28: Messung von Wellenleitereigenschaften in LB-Multischichten

Glasträger mit eingeätzten Gittern mit einem Gitterabstand $\lambda$ = 0,38 µm werden silanisiert und mit der Substanz 4 mit 50, 100 und 180 Doppelschichten bedeckt. Die Tauchrichtung stimmt mit der Richtung der Gitterlinien überein. Die Probe wird mit einem He-Ne Laser ($\lambda$ = 632,8 nm, 5 mW) beleuchtet. Dann wird der Film senkrecht zum Laserstrahl um die Tauchrichtung gedreht, bis Licht in den LB-Film eingekoppelt wird und Wellenleitung eintritt. Dies wird mit dem Lichtvektor parallel und senkrecht zur Tauchrichtung gemacht. Für den Lichtvektor parallel zur Tauchrichtung sind die Einkoppelungswinkel und die effektiven Brechzahlen $N_{eff}$ für die verschiedenen Schichtzahlen in Tabelle 10 zu finden.

Tabelle 1 Charakteristische Daten der hergestellten 2-Alkylamino-5-nitropyridine 1-6

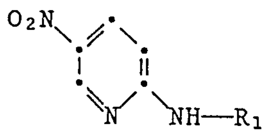

| Substanz | $R_1$ | Schmelzpunkt | C,H,N ber. | C,H,N gef. |
|---|---|---|---|---|
| 1 | $-C_{16}H_{33}$ | 76-78° | % C 69,38<br>% H 10,26<br>% N 11,56 | % C 69,33<br>% H 10,18<br>% N 11,47 |
| 2 | $-C_{18}H_{37}$ | 66-69° | % C 70,55<br>% H 10,55<br>% N 10,73 | % C 70,26<br>% H 10,43<br>% N 10,61 |
| 3 | $-C_{20}H_{41}$ | 80-82° | % C 71,55<br>% H 10,81<br>% N 10,01 | % C 71,43<br>% H 10,96<br>% N 9,92 |
| 4 | $-C_{22}H_{45}$ | 82-85° | % C 72,43<br>% H 11,03<br>% N 9,39 | % C 72,62<br>% H 11,07<br>% N 8,96 |
| 5 | $-C_{24}H_{49}$ | 85-88° | % C 73,21<br>% H 11,23<br>% N 8,83 | % C 73,32<br>% H 11,39<br>% N 8,57 |
| 6 | $-C_{26}H_{53}$ | 88-90° | % C 73,90<br>% H 11,40<br>% N 8,34 | % C 74,29<br>% H 11,59<br>% N 7,97 |

Tabelle 2: Charakteristische Daten der hergestellten Alkylamino-nitropyridine 7-10

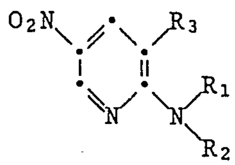

| Substanz | $R_1$ | $R_2$ | $R_3$ | Schmp. [°C] | C,H,N ber. | C,H,N gef. |
|---|---|---|---|---|---|---|
| 7 | $-C_{18}H_{37}$ | -H | $-NO_2$ | 74-77 | % C 63,27<br>% H 9,24<br>% N 12,83 | % C 63,45<br>% H 9,15<br>% N 12,84 |
| 8 | $-C_{18}H_{37}$ | $-C_{18}H_{37}$ | -H | 65-68 | % C 76,46<br>% H 12,05<br>% N 6,52 | % C 76,38<br>% H 11,97<br>% N 6,55 |
| 9 | $-C_{18}H_{37}$ | $-C_{18}H_{37}$ | $-NO_2$ | 36-41 | % C 71,46<br>% H 11,12<br>% N 8,13 | % C 71,25<br>% H 11,01<br>% N 8,26 |
| 10 | $-C_{22}H_{45}$ | $-CH_3$ | -H | 88-90 | % C 72,84<br>% H 11,13<br>% N 9,10 | % C 72,57<br>% H 11,09<br>% N 9,03 |

Tabelle 3: Charakteristische Daten der hergestellten Nitro-N-alkylaniline 11-17

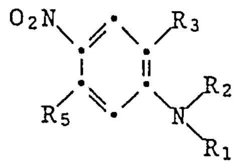

| Substanz | $R_1$ | $R_2$ | $R_3$ | $R_5$ | Schmp. [°C] | C,H,N ber. | C,H,N gef. |
|---|---|---|---|---|---|---|---|
| 11 | $-C_{18}H_{37}$ | -H | -H | -H | 78-80 | % C 73,80<br>% H 10,84<br>% N 7,17 | % C 73,75<br>% H 10,92<br>% N 7,10 |
| 12 | $-C_{18}H_{37}$ | -H | $-NO_2$ | -H | 58-59 | % C 66,17<br>% H 9,49<br>% N 9,65 | % C 66,15<br>% H 9,53<br>% N 9,59 |
| 13 | $-C_{18}H_{37}$ | $-C_{18}H_{37}$ | $-NO_2$ | -H | 40-42 | % C 73,31<br>% H 11,28<br>% N 6,11 | % C 73,13<br>% H 11,16<br>% N 6,09 |
| 14 | $-C_{18}H_{37}$ | -H | $-NO_2$ | $-NH_2$ | 97-99 | % C 63,97<br>% H 9,39<br>% N 12,43 | % C 63,81<br>% H 9,30<br>% N 12,29 |
| 15 | $-C_{22}H_{45}$ | -H | $-NO_2$ | $-NH_2$ | 108-110 | % C 66,37<br>% H 9,95<br>% N 11,06 | % C 66,36<br>% H 10,00<br>% N 11,02 |
| 16 | $-C_{18}H_{37}$ | $-C_{18}H_{37}$ | $-NO_2$ | $-NH_2$ | 63-66 | % C 71,75<br>% H 11,18<br>% N 7,97 | % C 71,60<br>% H 11,11<br>% N 7,95 |
| 17 | $-C_{22}H_{45}$ | $-CH_3$ | $-NO_2$ | $-NH_2$ | 87-89 | % C 66,89<br>% H 10,07<br>% N 10,76 | % C 66,32<br>% H 9,95<br>% N 10,84 |

Tabelle 4:

Charakteristische Daten der Monoschichten aus reinen Amphiphilen an der Gas/Wasser-Grenzfläche

| Beispiel Nr. | Substanz Nr. | Monoschichten Temp.d.Subphase [°C] | $F_c$ [mN/m] | $A_c$ [nm²/Mol] |
|---|---|---|---|---|
| 21a | 1 | 5 | 11 | 0,32 |
| 21b | 2 | 9 | 20 | 0,32 |
| 21c | 3 | 15 | 29 | 0,32 |
| 21d | 4 | 15 | 39 | 0,31 |
| 21e | 5 | 20 | 46 | 0,29 |
| 21f | 6 | 20 | 54 | 0,29 |
| 21g | 8 | 5 | 18 | 0,40 |
| 21h | 11 | 10 | 8 | 0,40 |
| 21i | 13 | 4 | 8 | 0,50 |
| 21j | 14 | 18 | 49 | 0,22 |
| 21k | 15 | 28 | 50 | 0,22 |
| 21l | 16 | 10 | 47 | 0,41 |

Tabelle 5

Charakteristische Daten der Monoschichten aus Amphiphil-Gemischen an der Gas/Wasser-Grenzfläche

| Bsp. Nr. | Substanzen | Molverhältnis in % | Monoschichten Temp. der Subphase [°C] | $F_c$ [mN/m] | $A_c$ [nm²/Mol] |
|---|---|---|---|---|---|
| 22a | Arachinsäure-methylester(ASME) (Fluka puriss.) | 33 | 20 | 24 | 0,26 |
| | und 4 | 67 | | | |
| 22b | ASME | 33 | | | |
| | und 4 | 67 | 15 | 31 | 0,24 |

Tabelle 6: Charakteristische Daten der LB-Filme aus reinen Amphiphilen

| Bsp. | Substanz | Multischichten | | | | | | | |
|---|---|---|---|---|---|---|---|---|---|
| Nr. | Nr. | $F_t$ [mN/m] | Temp. der Subphase T [°C] | Substrat | Tauchgeschw. $s_1$ [mm/Min.] | Tauchgeschw. $s_2$ [mm/Min.] | Zahl der übertragenen Doppelschichten | $d_{001}$ *) [nm] | $\lambda_{max}$ der Monoschicht [nm] |
| 23a | 2 | 9 | 9 | $C_{18}$-Glas | 5 | 10 | 15 | 3,77 | |
| 23b | 3 | 12 | 15 | $C_{18}$-Glas | 15 | 30 | 18 | 4,11 | 374 |
| 23c | 4 | 19 | 15 | $C_{18}$-Glas | 10 | 20 | 18 | 4,42 | 374 |
| 23d | 5 | 30 | 15 | $C_{18}$-Glas | 20 | 40 | 18 | 4,73 | 374 |
| 23e | 6 | 27 | 20 | $C_{18}$-Glas | 20 | 60 | 18 | 4,98 | 374 |
| 23f | 4 | 18 | 15 | $C_{18}$-Quarz | 10 | 20 | 270 | | |
| 23g | 5 | 24 | 20 | $C_{18}$-Quarz | 10 | 30 | 550 | 4,73 | |
| 23h | 4 | 18 | 15 | $C_{18}$-SiEinkris. | 10 | 20 | 18 | 4,36 | |
| 23i | 4 | 20 | 15 | ZnSe | 10 | 30 | 100 | | |
| 23j | 8 | 10 | 5 | $C_{18}$-Glas | 7,5 | 12,5 | 10 | 4,86 | 424 |
| 23k | 14 | 20 | 10 | $C_{18}$-Glas | 7,5 | 15 | 20 | 5,33 | |
| 23l | 16 | 18 | 15 | $C_{18}$-Glas | 12,5 | 25 | 20 | 3,91 | |
| 23m | 15 | 25 | 28 | $C_{18}$-Glas | 15 | 20 | 15 | | |
| 23n | 4 | 18 | 15 | $C_{18}$-Quarz | 15 | 30 | 5 | | 374 |
| 23o | 4 | 18 | 15 | $C_{18}$-Quarz | 15 | 30 | 10 | | 374 |
| 23p | 4 | 18 | 15 | $C_{18}$-Quarz | 15 | 30 | 15 | | 374 |
| 23q | 4 | 18 | 15 | $C_{18}$-Quarz | 15 | 30 | 30 | | 374 |

Fortsetzung Tabelle 6:

| Bsp. Nr. | Substanz Nr. | Multischichten $F_t$ [mN/m] | Temp. der Subphase T [°C] | Substrat | Tauchgeschw. $s_1$ [mm/Min.] | $s_2$ [mm/Min.] | Zahl der übertragenen Doppelschichten | $d_{001}$ *) [nm] | $\lambda_{max}$ der Monoschicht [nm] |
|---|---|---|---|---|---|---|---|---|---|
| 23r | 4 | 18 | 15 | $C_{18}$-Quarz | 15 | 30 | 45 | | 374 |
| 23s | 4 | 18 | 15 | $C_{18}$-Quarz | 15 | 30 | 60 | | 374 |
| 23t | 4 | 18 | 15 | $C_{18}$-Quarz | 15 | 30 | 90 | | 374 |
| 23u | 4 | 18 | 15 | $C_{18}$-Quarz | 15 | 30 | 135 | | 374 |
| 23v | 4 | 18 | 15 | $C_{18}$-Quarz | 15 | 30 | 400 | 4,37 | 374 |

*) Die Dicke einer Doppelschicht $d_{001}$ wird röntgenographisch unter Verwendung eines Philips Pulverdiffraktometers (Cu-$K_\alpha$-Strahlung) ermittelt.

Tabelle 7: Charakteristische Daten der LB-Filme aus Amphiphil-Gemischen

| Bsp. Nr. | Substanzen aus Beispiel Nr. (s. Tab. 5) | Multischichten $F_t$ [mN/m] | Subphasen T [°C] | Substrat | $s_1$ [mm/min] | $s_2$ [mm/min] | Zahl der Uebertragungsschichten | $d_{001}$*) [nm] | opt. Dichte pro Schicht (bei $\lambda_{max}$) |
|---|---|---|---|---|---|---|---|---|---|
| 25a | 22b | 18 | 15 | $C_{18}$-Glas | 20 | 60 | 18 | 5,16 | 0,0048 (370 nm) |

*) Die Dicke einer Doppelschicht $d_{001}$ wird röntgenographisch ermittelt (vergl. Tab. 6).

Tabelle 7

Charakteristische Daten der LB-Filme aus Amphiphil-Gemischen

| Bsp. Nr. | Substanzen aus Beispiel Nr. (s. Tab. 5) | Multischichten | | | $s_1$ | $s_2$ | Zahl der Uebertragungsschichten | $d_{001}$*) [nm] | opt Dichte pro Schicht (bei $\lambda_{max}$) |
|---|---|---|---|---|---|---|---|---|---|
| | | $F_t$ [mN/m] | Subphasen T [°C] | Substrat | [mm/min] | | | | |
| 25a | 22b | 18 | 15 | $C_{18}$-Glas | 20 | 60 | 18 | 5,16 | 0,0048 (370 nm) |

*) Die Dicke einer Dippelschicht $d_{001}$ wird röntgenographisch ermittelt (vergl. Tab. 6).

Tabelle 8

Frequenzverdopplung in LB-Schichtsystemen aus den reinen Amphiphilen 2-6 (vergl. Bsp. 27)

| Substanz | Zahl der übertragenen Schichten | $I^{2\omega}$ (relativ zu 4 ≡ 100) ∥* | ⊥* |
|---|---|---|---|
| 2 | 36 | 5 | 0,5 |
| 3 | 36 | 52 | 5 |
| 4 | 36 | 100 | 5 |
| 5 | 36 | 12 | 0,5 |
| 6 | 36 | 6 | 0,2 |

* Lichtvektor parallel bzw. senkrecht zur Tauchrichtung

Tabelle 9

| Zahl der übertragenen Doppelschichten | $\sqrt[2]{I^{2\omega}}$ (relative Einheiten)*) |
|---|---|
| 5 | 1,5 |
| 10 | 2 |
| 15 | 3,5 |
| 30 | 2,4 |
| 45 | 5,5 |
| 60 | 5,4 |
| 90 | 9,5 |
| 135 | 14 |
| 270 | 34 |

*) Die absolute Effizienz der Frequenzverdopplung wird an 270 Doppelschichten (siehe Text) ermittelt.

Tabelle 10

Einkoppelungswinkel und effektive Brechzahlen für Wellenleitung in LB-Multischichten von 4 für verschiedene Schichtdicken (Lichtvektor parallel zur Tauchrichtung) (vergl. Beispiel 28)

| Zahl der übertragenen Doppelschichten | Einkoppelwinkel [grad] | Effektive Brechzahl $N_{eff}$ |
|---|---|---|
| 50 | 9,65 | 1,503 |
| 100 | 9,03 | 1,514 |
| 180 | 8,84 | 1,517 |

**Patentansprüche**

1. Filmförmiges Material enthaltend Verbindungen der Formel I in orientierter, nicht punktsymmetrischer Anordnung

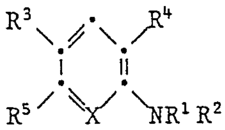

(I),

worin X =CH- oder =N- bedeutet, $R^1$ $C_{12}$-$C_{30}$-Alkyl ist, $R^2$ Wasserstoff oder $C_1$-$C_{30}$-Alkyl ist,
$R^3$ $-NO_2$, -CN, $-CF_3$, $-COCF_3$, $-SO_2CH_3$ oder $-SO_2CF_3$ bedeutet,
$R^4$ Wasserstoff ist oder die gleiche Bedeutung wie $R^3$ besitzt,
$R^5$ Wasserstoff oder $-NR^6R^7$ ist, und $R^6$ und $R^7$ unabhängig voneinander Wasserstoff oder $C_1$-$C_{30}$-Alkyl sind, wobei irgendwelche Alkylreste auch teil- oder perfluoriert sein können.

2. Material gemäss Anspruch 1, worin die Verbindungen der Formel I in Form von Langmuir-Blodgett Schichtsystemen (LB-Systemen) angeordnet sind.

3. LB-Systeme gemäss Anspruch 2 aus alternierenden oder nichtalternierenden Schichten enthaltend eine Verbindung der Formel I, worin X =CH- ist, $R^1$ $C_{18}$-$C_{22}$-Alkyl ist, $R^2$ Wasserstoff oder $C_{18}$-$C_{22}$-Alkyl bedeutet, $R^3$ $-NO_2$ ist, $R^4$ Wasserstoff oder $-NO_2$ bedeutet und $R^5$ Wasserstoff oder $-NH_2$ ist.

4. LB-Systeme gemäss Anspruch 2 enthaltend eine Verbindung der Formel I, worin X =N- ist.

5. LB-Systeme gemäss Anspruch 2 enthaltend alternierende oder nichtalternierende Schichten aus Verbindungen der Formel I, worin X =N-ist, $R^1$ $C_{16}$-$C_{26}$-Alkyl ist, $R^2$ Wasserstoff bedeutet, $R^3$ $-NO_2$ ist, $R^4$ Wasserstoff oder $-NO_2$ bedeutet und $R^5$ Wasserstoff ist.

6. Verbindungen der Formel I gemäss Anspruch 1, worin X =N- ist.

7. Verfahren zur Herstellung der Verbindungen der Formel I gemäss Anspruch 6, dadurch gekennzeichnet, dass man eine Verbindung der Formel IIa oder IIb mit der ein- oder zweifach stöchiometrischen Menge eines Amins der Formel III

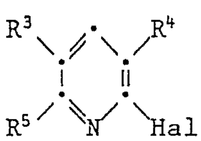

(IIa),

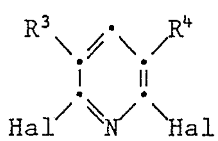

(IIb),

$HNR^1R^2$ (III),

worin $R^1$ bis $R^5$ die in Anspruch 1 definierte Bedeutung besitzen und Hal Chlor oder Fluor ist, umsetzt, so dass die Hal-Gruppen nukleophil gegen das Amin der Formel III ausgetauscht werden.

8. Verwendung der Verbindungen der Formel I gemäss Anspruch 1 zur Herstellung optoelektronischer Bauelemente, insbesondere als Frequenzverdoppler, Modulatoren oder als Wellenleiter.